# EUROPEAN PATENT SPECIFICATION

(11) **EP 0 867 721 B1**
(45) Date of publication and mention of the grant of the patent: **12.03.2003**
(21) Application number: 97302140.5
(22) Date of filing: 27.03.1997
(51) Int. Cl.: G01N 33/574, C12Q 1/16

(54) **Leukaemia detection method**
Verfahren zur Detektion von Leukämie
Procédé pour la détection de leucémie

(43) Date of publication of application: 30.09.1998
(73) Proprietor: Council of Scientific and Industrial Research, New Delhi 110 001 (IN)
(72) Inventor: Mandal, Chitra, Dr., Indian Inst.of Chem.Biology, Jadavpur, Calcutta - 700 032 (IN); Sinha, Diviya, Indian Inst. of Chem. Biology, Jadavpur, Calcutta - 700 032 (IN); Bhattacharya, Dilip Kumar, Department of Pathology, Calcutta 700 026 (IN)
(74) Representative: Walsh, David Patrick

(56) References cited:
- EP-A- 0 269 127
- WO-A-91/07424
- US-A- 5 330 897
- MOLECULAR AND CELLULAR BIOCHEMISTRY, vol. 136, 1994, pages 65-70, XP002040036 G. SEN AT AL: "O-acetylated sialic acid as a distinct marker for differentiation between several leukemia erythrocytes"

## Description

### FIELD OF INVENTION :

The present invention relates to a simple and effective method for the detection of minimal residual disease (MRD) and thereby identification or prediction of relapse by a lymphoproliferation assay in Acute lymphoblastic leukaemia (ALL). More specifically, the invention is for identification of a new key marker, namely, 9-O-Acetylated Sialoglycoconjugate with the help of a known 9-O-Acetylsialic acid binding lectin , AchatininH useful for the detection of minimal residual disease (MRD) and prediction of relapse by a lymphoproliferation assay in acute lymphoblastic leukaemia (ALL).

### BACKGROUND OF THE INVENTION :

The leukaemias are a heterogeneous group of neoplasms arising from the malignant transformation of heamatopoietic i.e. blood forming cells. Leukaemia can be broadly classified accordingly to the cell type involved primarily (myeloid or lymphoid) and as acute or chronic depending on the natural history of the disease.

Acute lymphoblastic leukaemia (ALL) is the commonest type of leukaemia in childhood. It is a primarily a disease of children and young adults. It occurs in all races with a peak incidence in children between 3 to 5 years of age. Acute lymphoblastic leukaemia (ALL) is diagnosed in 2000 to 3000 new cases of childhood leukaemia in United States each year, whereas Acute myelogenous leukaemia (AML) is diagnosed in only 500 children and Chronic myeloid leukaemia (CML) in fewer than 100. About 40 million children in U.S.A are affected under the group of 15 years, about 3/4 Th. of these have ALL. (Nelson essential of Paedratics. 1990 Ed Bchrman RE and Klregman R pp.53).

The causes are not known, but environmental agents including irradiation, chemical carcinogens, cytogenetic abnormalities and retrovirus infections are known to play an important role in the etiology of leukaemia. For instance, individuals with occupational accidental radiation exposure, patients receiving radiation therapy or survivors of the atomic bomb explosions in Japan have a predictable and dose related increased incidence of leukaemia.

Pediatric haematopoietic malignancies rank first in cancer incidence and mortality in children and are responsible for roughly *40 %* of childhood related death (Carp and McCaffrey J. Natl. cancer. Inst. 86, 1196, 1994).

In fact, the diagnosis and the treatment of Acute lymphoblastic leukaemia (ALL) is one of the major success stories of modem clinical oncology. For oncologists, Acute lymphoblastic leukaemia (ALL) represents a major therapeutic success as remission can be achieved in nearly 65 % of patients(Pui and Crist Curr. Opinion in Oncology 7, 36,1995, Lancet 347, pp 1783, 1996). With the available chemotherapeutic treatment, most of the Acute lymphoblastic leukaemia (ALL) patients can lead a normal disease free life for approximately 3-5 years. However, relapse and eventual treatment failure invariably occurs in most cases receiving identical treatment and this area is a major challenge for leukaemia specialists (Pui and Crist N. Eng. J. Med. 332, 1618,1995). The reason behind the occurrence of relapse can be well understood if we take into consideration the definition adopted by the clinicians to state whether the patient has attained complete remission or not. The clinicians state :-
a. If the laeukemia blasts in the bone marrow of the patients is less than 5% and
b. If no laeukemia blast is detected in the peripheral blood of patient, the patient is said to be in the state of apparently complete remission.

Therefore, the flaw lies in the definition itself since it states that leukaemic blast cells within a range of 0-5% may still be present within the patient when he is said to have attained the so called complete remission. At the time of diagnosis, the leukaemic cell mass is usually between 10¹¹ - 10¹² cells and available chemotherapeutic agents produce a fractional cell kill capable of a 3 to 5 log kill resulting in the elimination of 99.99 to 99.999 % of leukaemia cells. The remaining 0.01 to 0.001% leukaemic cells tantamounts to the persistence of 10⁸ to 10⁹ leukaemic blast cells respectively (Champlin and Golde Harrison Text book of Internal Medicine, 1552). What is important is that these persisting leukaemic blast cells are not detectable by standard morphology in bone marrow or peripheral blood. It is these cells that are responsible for relapse, if post induction chemotherapy fails to eradicate them. To eliminate this non-detectable yet existing leukaemic cell mass, maintenance therapy is given for an extended period( 2 to 2.5 years) with the purpose of reducing the possible relapse and possibly eradicating this "iceberg" like leukaemic mass.

These residual laeukemia blasts remaining in the patient, which cannot be detected by the available techniques comprise the minimal residual disease (MRD) i.e. described as Phase B, C and D in results. It is the proliferation and infiltration of these blast cells which serves as the major cause of relapse. This "iceberg" is conventionally addressed as minimal residual disease (MRD) (Knechtli et. al. J. Clin Path 48,1995 ). It is defined as the presence of leukaemic cells not detectable by morphology . Assays to detect minimal residual disease (MRD) is the need of the hour as these will help the clinicians to assess the effect of treatment on tumour burden and allow anticipation of relapse with greater precision (Brisco, Condan, Hughes, et.al. The Lancet 1994 ).

Therefore, the applicants have developed a simple blood based lymphoproliferative assay to detect minimal residual disease (MRD) and predict relapse in Acute lymphoblastic leukaemia (ALL) patients employing AchatininH as the tool.

### PRIOR ART REFERENCES:

Currently no specific marker is available to pin point the dose of chemotherapy and duration of maintenance therapy in acute lymphoblastic leukaemia (ALL).

Existing methods for the detection of leukaemic blasts cells are i) cytomorphology and karyotyping ii) immunological methods and iii) molecular detection :
**Cytomorphology and karyotyping :** Acquired non-random chromosomal translocation occurs in 30 -70 % of Acute lymphoblastic leukaemia (ALL) patients and can serve as marker of this disease. But the approach has limited sensitivity (1 to 5% ) primarily due to the paucity of leukaemic blast cells during clinical remission (Campana D Pui Blood 85, 1416,1995). Fluorescent in situ hybridization using chromosome specific or locus specific probes allows to identify abnormal ties in cells at metaphase (Leo Beau Blood, 81,1979,1993). Cytomorphology and Karyotyping techniques can detect one laeukemia blast in total population of 100 cells. Therefore they are said to have a sensitivity of 1% or 10⁻².
**Immunological methods :** Immunological methods based on the recognition of leukaemia associated phenotypes not usually found in normal bone marrow have had promising results (Cole et. al. Baillieres Clin Haematol 7, 183, 1994). Immunophenotyping has been complemented by flow cytometric analysis where a combination of markers have been able to quantify minimal residual disease (MRD).

### Main disadvantages:

1. Flow cytometric analysis can detect one laeukemia blast cell in a total cell population of 1000. So it is said to have a sensitivity of 0.1% or 10⁻³, which is inferior to available DNA based method (Huh and Andreef 8, 713,1994, Meydan et al. Nature 379, pp 645, 1996).
2. The flow cytometre used for Flow cytometric analysis is very costly (about U.S. $1500) and not available in clinics.
3. It requires mandatory technical expertise.
4. The method needs expensive chemicals e.g. several specific antibodies and fluorescence conjugated chemicals.
**Molecular approaches :** In the majority of cases, relapse of acute lymphoblastic leukaemia is thought to involve the same leukaemic clone as the original disease (Bunin et.al. Leukaemia, 4, 722,1990). Around 80 % of cases of childhood acute lymphoblastic leukaemia are due to clonal expansion of precursor B cells and have rearrangement of IgH gene, from which specific DNA probes have been generated. Several PCR based methods (Brisco M J. Condan J, Hughes E, et al. The Lancet 19945 ; 343: 196-200, Veelken H, Tyeko B, Sklar J.Blood 1991 : 78 : 1318-1326, Wasserman R, Galili N, Ito J,et al.J of Clin. Oncol. 1992; 10 : 1879 -1880) have been reported for the detection of MRD in childhood acute lymphoblastic leukaemia (ALL).

These methods are based on the extraction of DNA from marrow and PCR amplification of tumour specific DNA or RNA sequences. The immunoglobulin heavy chain gene is used as a molecular marker to quantify leukaemic blast cells and N- ras gene to quantify total marrow cells. The number of amplifiable leukaemia and N-ras genes per ng of marrow DNA is calculated from Poisson's statistics and the level of minimal residual disease (MRD) is calculated from the ratio of the two heavy chains genes in each leukemia cell and 2N- ras genes in each marrow cell. However, these published methods are successful in only half of the patients since different individuals can show different rearrangement of immunoglobulin genes or T cell Receptor genes.

### The principle Drawbacks of the PCR methods for routine follow up of the MRD

Recently, the PCR methods have been developed to quantitate minimal residual disease (MRD) which detect the rearranged immunoglobulin or TCR genes in leukaemia cells (Brisco et al., 1994 ; Kumar., 1995). Employing the polymerase chain reaction, it has been shown that even one malignant cells per 10⁵ normal cells can be detected (O' Reilly et al., 1995). If both immunoglobulins and the T cell receptor gene rearrangements are studied. PCR has the potential to detect minimal residual disease (MRD) in nearly 90% of the cases for approximately one year despite clinical remission (Ito et al., 1994). However, technical and biological problems related to PCR exist, namely:
(i) occurrence of false positives due to contamination of reaction mix with previously employed samples
(ii) occurrence of false negatives results owing to degraded RNA or DNA or clonal evolution in approximately 20% of cases (Pui., 1995)
(iii) not all laeukemia specific IgH and TCR gene rearrangements are amenable to initial amplification of PCR using universal primers
(iv) a heterogeneous distribution of minimal residual disease (MRD) may result in sampling error since IgH and TCR gene rearrangements may be different in different individuals.
(v) these methods are costly, lengthy, very sophisticated and requiring mandatory technical expertise.

Since PCR undetectable residual disease is necessary for cures in most patients, it can be proposed that molecular remission defined as PCR undetectable disease is a milestone and target for achieving cure.

Evidently all these problems are unlikely to be exploited for commercial basis.

In India, the research in the field of acute lymphoblastic leukaemia (ALL) is mainly carried out at the Tata Memorial Hospital, Bombay. Their interest in acute lymphoblastic leukaemia is focused on cytogenesis (Gladstone et. Al. Ind. J. Med. Res. 99, 264,1994). Infection analysis (Raje et.al. Pediatr. Hematol. Oncol. 11, 271, 1994) and central nervous system relapse (Iyer et. al. Leuk. Lymphoma, 13, 183, 1994).
Recently, a paper entitled 'O-acetylated sialic acid as a distinct marker for differentiation between several leukaemia erythrocytes.' Molecular and cellular Biochemistry 136 : 65-70 ; 1994 mentions that a lectin (Achatinin H) isolated from the hemolymph of *Achatina fulica* snail, which has been shown to have narrow specificity towards 9-O-acetyl sialic acid and this paper deals with identification 0f 9- O- acetylated glycoconjugates on erythrocytes only and nothing beyond. In addition, with reference to this publication of 1994, the following points must be taken into consideration :-
1. As stated by the title, the paper identifies O-acetylated sialic acid as a distinct marker for differentiation between several leukemia erythrocytes only and not specifically to Acute lymphoblastic leukaemia (ALL). Therefore, it deals with the identification of O acetylated glycoconjugate on erythrocytes only.
2. The paper summarizes the result of a study of binding of AchatininH with erythrocytes of patients suffering from Acute lymphoblastic leukemia (ALL) in their acute phase only.
   Moreover, this paper does not describe or even envisage any experiment being performed with Peripheral blood mononuclear cells (PBMC) of these patients and accordingly had not addressed the problem of minimal residual disease and prediction of relapse.
3. In the above referred paper, very few patients (only five Acute lymphoblastic leukaemia (ALL) patients) have been included in the study and hence the result should not be considered as statistically significant.
4. In the above referred paper, the method is hemagglutination assay with erythrocytes but not the lymphoproliferation assay with peripheral blood mononuclear cells.
5. The study includes newly diagnosed Acute lymphoblastic leukaemia (ALL) patients who have not received any chemotherapeutic treatment, Thus, the preliminary work was only with the acute phase of the disease This is nothing to do with the detection of minimal residual disease (MRD).
6. The hemagglutination assay with erythrocytes has also been performed with Acute lymphoblastic leukaemia (ALL) patients at different stages of treatment, i.e. Phases A,B,C,D and E no agglutination could be observed.

This clearly reflects that 9- O- acetylated glycoconjugates is transiently expressed on erythrocyte cell surface of only untreated Acute lymphoblastic leukaemia (ALL) patients (positive hemagglutination) but completely disappears with onset of treatment (no hemagglutination). Therefore, the transient expression of this 9- O- acetylated glycoconjugates on erythrocytes can not to predict minimal residual disease (MRD).

On the other hand, the present invention refers to the identification of 9-O-acetylated sialoglycoconjugates on peripheral blood mononuclear cells (PBMC) of children suffering from acute lymphoblastic leukaemia and not on their erthyocytes, detection of minimal residual disease in acute lymphoblastic leukaemia and prediction of relapse in acute lymphoblastic leukaemia, and the above features cannot be envisaged in the above publication.

An extensive world wide patent search has not shown any patent which claims that they can detect minimal residual disease (MRD) in Acute lymphoblastic leukaemia (ALL) using peripheral blood.
Presently patents filed in the area of diagnosis of leukaemias focus mainly on polymerize chain reaction methods namely :-
1. Detecting minimal residual disease - in lymphoid malignancies using the polymerase chain reaction. Drawbacks are a) It is specific for lymphoid malignancies i.e. chronic and acute lymphoblastic leukaemia and not just ALL and it is PCR based.
2. BCL - 3 gene sequences -used to detect cellular oncogenic C-myc translocation, leukaemia (s), and to monitor anti-neoplastic therapy Drawbacks are a) It is not specific for acute lymphoblastic leukaemia but any neoplastic transformation resulting due to C-myc oncogenic expression and is PCR based. Moreover detection of minimal residual disease (MRD) is not mentioned as per the method.
3. DNA encoding human common acute lymphoblastic leukaemia antigen-used for obtaining pure protein for diagnosis and treatment of medical conditions. Drawbacks are the following a) The method is PCR based and detection of MRD is not mentioned as per the method.

Considering the drawbacks of the PCR methods as described in the prior art, development of a diagnostic assay which is simple, sensitive and specific is urgently required to detect and quantify Minimal residual disease (MRD) to control Acute lymphoblastic leukaemia (ALL) related sufferings.

To the best of our knowledge, there is presently no group. national or international who are studying detection and quantification of MRD employs the lectin AchatininH as a probe using peripheral blood. The selectivity of this lectin AchatininH has allowed not only the identification but also quantification of this residual blast or cancer cells using peripheral blood.

Sialic acids are a family of derivatives of N-acetyl or N-glycolyl neuraminic acids and are very important constituents of cell surface architecture. Sialic acids play an important role in receptors for virus, peptide hormones and toxins (Rosenberg A and Schengrund CL. Plenum Press, New York 1976). Sialic acids also functions as making agents on antigens, receptors and other recognition sites of the cell surface (Burness ATH. Animal Viruses. Chapman and Hall, New York 1981: Varki A Glycobiology, 2, pp25, 1992). The O substituted sialic acids exhibit species and tissue specific distribution in animals (Schauer R adv. Carbohydr. Chem. Biochem. 1982 : 40; 131). Changes in sialic and the degrees of O-acetylation of sialic acid residues have been reported in transformed and malignant cells.

Detection of sialic acid residues can be approached through the use of sialic acid specific lectins found in a variety of invertebrates (Mandal C and Mandal C Experientia 1990 : 46 ; 433 - 441 ; Yeaton RW Dev. comp. Immun. 1981 : 5 ; 391) and serum (Tsai CM, Zopf DA,Yu RK, wister R Jr., Ginsberg V Proc. Natl. Acad Sci. USA 1977 : 74 ; 4591 -4594).

AchatininH is a sialic acid binding lectin. This lectin has been prepared by allowing hemolymph to clot at room temperature, centrifugation for, seperation of cells from the supernatant liquid, dialyzing using a known buffer containing calcium at a pH in the range of 8 and 9 and temperature in the range of 10 -25°C, passing the dialyzed hemolymph through an affinity column containing a glycoprotein, which will bind the lectin contained in the dialyzed hemolymph, washing the column using a buffer to remove unwanted protein from the column and elution of the bound protein with a special buffer without calcium at a pH 8 to 9. This process has been described in our patent No 165730, Basu S, Sarkar M, Mandal C. Molecular and Cellular Biochemistry 1986 ; 71 : 149-157). The lectin binds preferentially to sialic acid derivatives which are : (a) O-acetylated at the C-9 position of the parent molecule and (b) have an α 2,6 linkage (Mandal C and Basu S. Biochem. Biophys Res comm 1987 ; 148 : 795-801, Sen G, Chowdhury M and Mandal C. Carbohydrate Research 1995, 268 : 115-125 ). The selectivity of this lectin has been used for the study of ALL. This allowed us to identify a key marker (9-O-Acetyl Sialoglyco conjugate) on the PBMC surface which helped us to develop a rapid accurate and inexpensive process for diagnosis, detection of minimal residual disease (MRD) and prediction of relapse in Acute lymphoblastic leukaemia (ALL).

Sialic acid residues are commonly O-acetylated at the C-4, 7, 8 and 9 position of the parent molecule. Of these, 9-O acetylation occurs in very small amounts on normal lymphocytes and more importantly this 9-O acetylated sialoglycoconjugate is absent on normal human erythrocytes (Schauer R Adv. Carbohydr. Chem Biochem.1982 : 40 ; 131-234 ). Accordingly, we have exploited the selective binding of AchatininH to 9- O acetylated derivatives (9-O AcSG) to serve as a diagnostic tool for detecting specific transformations on PBMC surface involving this biomarker. It has allowed us to identify the presence of 9-O-AcSG on the cell surface of blast cells of the children suffering from acute lymphoblastic leukaemia (ALL). specially during maintenance therapy using a 9-O acetylated sialic acid binding lectin AchatininH.

The presence of 9-O-AcSG has been corroborated by flurometric quantitative analysis of 9-O-acetylated sialic acid on the surface of PBMC.

### SUMMARY OF THE INVENTION

Therefore, the invention provides an identification of a new key marker, namely, 9-O-Acetylated Sialoglycoconjugate with the help of a known 9-O-Acetylsialic acid binding lectin , AchatininH useful for the detection of minimal residual disease (MRD) and prediction of relapse by a lymphoproliferation assay in acute lymphoblastic leukaemia (ALL).

### OBJECTS AND DETAILED DESCRIPTION OF THE INVENTION :

The main object of the present invention is to provide a simple and effective detection of Minimal Residual disease in Acute lymphoblastic leukaemia and thereby identifying or prediction of relapse by a lymphoproliferation assay.

Another object of the invention provides a process for identification of a new key marker, i.e. a cell surface receptor, namely, a 9-O-Acetylated Sialoglycoconjugate with the help a known 9-O-Acetylsialic acid binding lectin AchatininH useful for the diagnosis of acute lymphoblastic leukaemia (ALL). detection of minimal residual disease (MRD) and prediction of relapse by a lymphoproliferation assay.

Yet another object of the present invention is to provide a process for the determination of a receptor on the cell surface of blast cells of the children suffering from acute lymphoblastic leukaemia (ALL) during maintenance therapy by a simple, specific, sensitive, non-invasive and economical, semi-automated colorimetric assay method which obviates the drawbacks as detailed above.

The proposed assay will allow for the assessment of the number of leukaemic blast cells either by a radioactive thymidine uptake lymphoproliferative assay method or by a simple, specific, sensitive, non-invasive and economical, semi-automated colorimetric assay method specially during maintenance therapy when the number of leukaemic blast cells are very low in the blood (less than 5 %) and can not be detected by conventional methods.

Still another objective of the present invention is to provide a process for the quantification of leukaemic blast cell surface receptor which will reflect number of the leukaemic blast cells present in these children at different clinical stages of the disease. So, this invention provides an indicator as to when and how long should chemotherapy be continued. The process can predict the probability of relapse. So this invention will serve as an effective tool in the battle for identification of minimal residual laeukemia blast cells i.e. Minimal Residual Disease in children suffering from acute lymphoblastic leukaemia.

To meet the above objects, the present invention provides a simple, easy, sensitive, specific, effective and inexpensive method for the detection of MRD and prediction of relapse by identifying a key marker, i.e. cell surface receptor -9 O-acetylated sialoglycoconjugate" on the surface of peripheral blood mononuclear cells of acute lymphoblastic leukaemia patients. said method comprising collecting blood from acute lymphoblastic leukaemia patients at different clinical states of the disease ; separating peripheral blood mononuclear cells by centrifugation ; culturing them in tissue culture medium with AchatininH, harvesting the mononuclear cells and counting such cells and thereby diagnosing the disease, or such cultures were pulsed with 3-(4,5-dimethylthiazole-2-yl)-2,5-diphenyltetrazolium bromide (MTT) for 4 hours before the termination of culture at 37°C, adding a solvent DMSO to dissolve the purple formazan crystals at the end of the incubation, and measuring the optimal density at 560 nm and 690 nm reference in an ELISA reader, or the level of residual leukaemia cells (MRD) is determined by the optimal concentration of required for maximal proliferation.

Accordingly, the present invention provides an identification of a new key marker, namely, 9-O-Acetylated Sialoglycoconjugate with the help of a known 9-O-Acetylsialic acid binding lectin , AchatininH useful for the detection of minimal residual disease (MRD) and prediction of relapse by a lymphoproliferation assay in acute lymphoblastic leukaemia (ALL) and by a simple, specific, sensitive, non-invasive and economical, semi-automated colorimetric assay method. The applicants have designated this cell surface receptor as "9 O-acetylated sialoglycoconjugate" useful to reliably detect and quantify the small number of residual blast cells i.e. MRD, which comprises. I) collection of heparinised blood from acute lymphoblastic leukaemia patients (ALL) at different stages of the disease ii) separation of peripheral blood mononuclear cells (PBMC) by Ficoll Hypaque density centrifugation iii) seeding in triplicate at a density of 1 x 10⁵ cells /well in RPMI 1640, supplemented with glutamine, penicillin, streptomycin and 10 % heat inactivated human AB serum iv) culturing with 0.05 -10 µg of the 9-O acetylated sialic acid binding lectin, AchatininH in 96 well flat bottom micortitre plates in a total volume of 250 µl, at 37°C in a humidified atmosphere of 5 % CO2 and 95 % air/v ) After 4 days, cultures were pulsed with ³H Thymidine, 18 hours before the termination of culture . Cells were harvested and counts were taken. Alternatively, pulsing the proliferative cells were performed by using 3-(4,5-dimethylthiazole-2-yl)-2,5-diphenyltetrazolium bromide (MTT).

In the present process, the whole blood is used for peripheral blood mononuclear cells operation and the 9-O- acetyl sialic binding lectin. Achatinin H, purified from the hemolymph of *Achatina Fulica* snail was used as a unique reagent for stimulation of peripheral blood mononuclear cells in all experiments. Preferably, the concentration of AchatininH is used in the range of 0.05-10µg for stimulation of peripheral blood mononuclear cells.

The culture medium used in the present process is selected from RPMI 1640, supplemented with glutamine, antibiotics and 10 % heat inactivated human AB serum or another medium such as DMEM and fetal calf serum and the culturing of the peripheral blood mononuclear cells is conducted in 96 well flat bottom microtitre plates, Tarson, Nunc, Costor or corning plates and sterile test tubes.

In a preferred aspect, 0.25µl to 0.5 ml of a total reaction volume is used to culture peripheral blood mononuclear cells, the peripheral blood mononuclear cells are being cultured at 37°C in the presence of AchatininH and the peripheral blood mononuclear cells are being cultured in the presence of AchatininH in a humidified atmosphere of 5% CO₂ and 95 % air.

In addition, the culture time of 4 days is used for maximum stimulation and the peripheral blood mononuclear cells concentration of 1x 10⁵ cells/well are giving best stimulation. Further, the cultures are regularly pulsed with 37 kBq (1µCi) of ³H thymidine or 1.85 kBq (0.05µCi) of ³H thymidine, 37 kBq (1µCi) of ³H thymidine was added 18 hours before the termination of culture and 3-(4,5-dimethylthiazole-2-yl)-2,5-diphenyltetrazolium bromide (MTT) is added 4 hours before the termination of culture at 37°C. At the end of the incubation, solvent such as DMSO or SDS is added to dissolve the purple formazan crystals and the optical density is measured at 560 nm with 690 nm reference in an ELISA reader. The amount of formazan produced which is proportional to the number of viable cells after cell proliferation is estimated colorimetrically and the dose of AchatininH (µg/1x10⁵ cells) needed for maximum proliferation of peripheral blood mononuclear cells is calculated.

The dose of AchatininH(µg/1x10⁵ cells) needed for maximum proliferation of peripheral blood cells been correlated with the status of the disease, increasing with progress of chemotherapy. The level of minimal residual leukaemia cells (MRD) is determined by the optimal concentration of AchatininH required for maximal proliferation.

To make the lymphoproliferative assay more rapid & convenient, conventional ³H thymidine uptake has been replaced by 3-(4,5-dimethylthiazole-2-yl)-2,5-diphenyltetrazolium bromide (MTT). 3-(4,5-dimethylthiazole-2-yl)-2,5-diphenyltetrazolium bromide (MTT) was added before the termination of culture at 37°C followed by incubation. A solvent was added to dissolve the purple formazan crystals. The optical density was measured at 560 nm with 690 nm reference in an ELISA reader.

In order to clearly state the lymphoproliferative data, the design and the hypothesis behind the lymphoproliferative assay is stated in great details as follows:-
(i) Peripheral blood mononuclear cells (PBMC) of Acute lymphoblastic leukaemia (ALL) patients, patients of other hematological disorders (AML, CML, NHL and Thalassemia) and normal donors were isolated by Ficoll Hypaque density centrifugation.
(ii) PBMC (1x10⁵cells/well) were cultured with different concentrations of Achatinin_{H} (0.05-10ug) in Medium A for 96 hours at 37°C with 5% CO₂ and 95% air.
(iii) The cultures were pulsed with ³H-Thymidine (37 kBq/well; 1µCi/well) for 18 hours.
(iv) The cells were harvested and counts taken in Rack Beta LKB liquid scintillation counter.
(v) For each patient and normal donor, the counts obtained were plotted in y axis against the Achatinin_{H} dose plotted on x axis which ranged from 0.05-10ug.
(vi) From this plot, the dose of Achatinin_{H} at which maximal incorporation of radioactivity is obtained was determined. This has been designated as the maximal lymphoproliferative dose of Achatinin_{H} for that patient and normal donor.
(vii) Accordingly, the mean of the maximal lymphoproliferative dose of Achatinin_{H} for Acute lymphoblastic leukaemia (ALL) patients has been determined and compared with normal donors.

### Interpretation of the lymphoproliferative assay.

In a generalized way mitogen is any substance which stimulates cellular proliferation. A mitogen may be classified as potent for a particular cell type if,
(i) Maximal proliferative dose of mitogen is very low and
(ii) the density of the cell surface receptor to which the mitogen binds for mediating cellular proliferation is very high.

### BRIEF DESCRIPTION OF THE ACCOMPANYING DRAWINGS

The results of all the phases of the patients Acute lymphoblastic leukaemia (ALL) and the percentage of recovery of individual patients in response to treatment were determined.

The dose of Achatinin_{H} (X+ SD ug/1x10⁵ cells) required for maximal lymphoproliferation of Acute lymphoblastic leukaemia (ALL) patients at different phases of treatment was determined.

In Phase A (n = 65, Male : Female = 52 : 13), Achatinin_{H} dose for maximal lymphproliferation was 0.19 + 0.019ug. Details of the individual patients data has been presented in Table 1.

Maximal lymphoproliferative dose of Achatinin_{H} for Acute lymphoblastic leukaemia (ALL) patients is increased to 1.34+ 0.13 ug in Phase B (n = 27, Male : Female = 24:3). The table 2 describes the individual patients in phase B.

Maximal lymphoproliferative dose of Achatinin_{H} for Acute lymphoblastic leukaemia (ALL) patients is further increased to 5.63 + 0.30 ug in Phase C (n = 35, Male : Female = 29 : 6) as described in Table 3.

Table 4 describes the individual patients of follow up studies and the maximal lymphoproliferative dose of Achatinin_{H} for these patients is increased to 6.18 + 0.32 ug in Phase D (n = 11, Male) compared to acute phase i.e. Phase A..

On contrary, with relapse (Phase E, n = 8, Male : Female= 6 : 2) maximal stimulatory dose of Achatinin_{H} showed a sharp decline comparable to Phase A as predicted in Table 5.

Five different blood related disorders (NHL, CML, AML, Thalassemmia and Aplastic anemia) served as negative controls showing no cross reactivity with Acute lymphoblastic leukaemia (ALL) by this invented process (Table 6).

PBMC of normal donors required an Achatinin_{H} dose of 7.92 + 0.08 ug (n = 25) for maximal lymphoproliferation (please see Table 7).

The results of all the phases of the patients Acute lymphoblastic leukaemia (ALL) were determined. From this study reflects a variation in the Achatinin_{H} concentration for maximal lymphoblastic response pointing towards the differential expression of this key marker, a "9-O-acetylated sialoglycoconjugate", at different clinical stages of Acute lymphoblastic leukaemia (ALL). During Phase A, the key receptor is optimally expressed as shown by the lower Achatinin_{H} concentration for maximal lymphoproliferation. With progressive chemotherapy, either the expression of the receptor shows a marked decline or there occurs a masking of 9-O-acetyl sialic acid moieties of this receptor as evident from the increase in required dose of Achatinin_{H} for maximal lymphoproliferation. However, the Achatinin_{H} dose for maximal lymphoproliferation shows a marked decline following relapse comparable with Phase A, reflecting the re-expression of this marker with relapse. Hence, the Achatinin_{H} dose for maximal lymphoproliferation bears an inverse relationship with peripheral blast cell % and % Recovery of individual patients in response to treatment thereby serving as a reflection of Minimal Residual Disease (MRD).

Since the maximal lymphoproliferative dose of Achatinin_{H} for ALL patients is significantly low in comparison to normal donors, we infer that mitogenic potential of Achatinin_{H} in Acute lymphoblastic leukaemia (ALL) patients is significantly higher than that for normal donors. Moreover, since Achatinin_{H} is a 9-O Acetyl sialic acid binding lectin lymphoproliferative assay serves as an indirect measure of increased "9-O-acetylated sialoglycoconjugate" expression on PBMC surface of ALL patients in comparison to normal donors.

Therefore, the applicants identified that the 'maximal lymphoproliferative dose' of Achatinin_{H} bears an inverse relationship with the expression of "9-O-acetylated sialoglycoconjugate" key marker on PBMC surface. Therefore the expression of this key marker serves as a direct reflection of laeukemia blast cells i.e. minimal residual disease (MRD) in Acute lymphoblastic leukaemia (ALL) patients and can predict relapse.

Thus the amount of AchatininH needed for maximum stimulation of peripheral blood mononuclear cells was determined. This allowed determination of the unique cell surface receptor, namely "9-O-acetylated sialoglycoconjugate" which could be exploited to reliably detect and quantify the small number of residual blast cells i.e. MRD which stay in peripheral blood even after chemotherapy. So, it can serve as an index to determine the dose and duration for which maintenance therapy should be continued to achieve complete cure. Thus its correlation with the status of the disease was assessed.

In a preferred embodiment of the invention, following are the possible permutations and combinations of the above mentioned process :

Whole heparinised blood is used for peripheral blood mononuclear cell separation. Alservers solution or any anticoagulant can be used for collection of blood. The concentration of AchatininH can vary in the range of 0.1 to 10 µg in 0.85 % saline or tissue culture medium. Others buffers such as This hydrochloride buffer, can also be used. 96 well U bottom microtitre plates are used for lymphoproliferation assay. 96 well V shaped microtitre plates can also be used. Sterile Tarson (indigenous) or Nunc or Coastor or coming plates are equally good. Small glass or plastic tubes also can be used. The total reaction volume is 0.25 ml for lymphoproliferation assay. The volume can be varied from 0.25 µl - 0.5 ml for lymphoproliferation assay. Peripheral blood mononuclear cell have been incubated at 37°C in presence of AchatininH. The temperature can be varied from 30 °C to 38°C. Whole reaction was carried out in presence of 30 mM of Ca²⁺. However, the concentration of Ca²⁺ can be varied from 25 to 30mM. The reaction time of 96 hours was used for maximum proliferation. The time may be varied from 24 to 72 hours. The amount of AchatininH needed for maximum proliferation did not change even if the cells were allowed to proliferate only for 24 hours. However, a reduction in the count was observed. Peripheral blood mononuclear cells (1x 10⁵) were cultured in triplicates. The number of peripheral blood mononuclear cells can be varied from 1x10³ to 1x10⁵ cells. However, culture time of 96 hours and cell concentration 1x10⁵ gave best lymphoproliferation. Radioactive thymidine can be used at 1.85 or 37 kBq (0.05 or 1µCi). Instead of radioactive thymidine, culture can be pulsed with MTT 4 hours before the termination of culture at 37°C. At the end of the incubation, a solvent, DMSO, is added to dissolve the purple formazan crystals. Any other solvent e.g. sodium dodecyl sulphate or combination of sodium dodecyl sulphate and hydrochloric acid or dimethyl sulfoxide or proponal or ethanol can be used to dissolve the purple formazan crystals. The optical density is measured at 560 nm with 690 nm reference in an ELISA reader to assess the lymphoproliferation.

The following examples are given by a way of illustration of the present invention and these should not be contrued to limit the scope of the present invention :

### Example 1

Approximately 2-3 ml of blood was collected from acute lymphoblastic leukaemia patients (n=40). Peripheral blood mononuclear cells were separated by Ficoll Hypaque density centrifugation and seeded at a density of 1x10⁵ cells/well in RPMI 1640, supplemented with glutamine, penicillin, streptomycin and 10 % heat inactivated human AB serum. Cell viability was more than 95 % as checked by their ability to exclude Trypan blue dye(0.2 %). Peripheral blood mononuclear cells were cultured in triplicates(1x10⁵ cells/well) with different concentration of AchatininH (0.05 to 10µg ) in 96 well flat bottom microtitre plates: in a total volume of 250µl , at 37°C in a humidified atmosphere of 5 % CO₂ and 95 % air .A culture time of 4 days and cell concentration of 1x10⁵ cells/well showed best results. Cultures were pulsed with 37 kBq (1µCi) of ³H Thymidine, 18 hours before the termination of culture. Cells were harvested and counts were taken.

### Example 2

Approximately 2-3 ml of blood was collected from acute lymphoblastic leukaemia patients (n=50). Peripheral blood mononuclear cells were separated by Ficoll Hypaque density centrifugation and seeded at a density of 1x10⁴ cells/wells in RPMI 1640 (it is a trade name), supplemented with gultamine, gentimycine and 10 % heat inactivated human AB serum. Cell viability was more than 95 % as checked by their ability to exclude Trypan blue dye (0.2.%). Peripheral blood mononuclear cells were cultured in triplicates (1x10⁴ cells/well) with different concentration of AchatininH (0.05 -10µg) in 96 well flat bottom microtitre plates in a total volume of 250µl, at 37°C in a humidified atmosphere of 8% C02 and 95% air. Cultures were pulsed with 37 kBq (1µCi) of ³H Thymidine, 18 hours before the termination culture after culturing the cells for 3 days. Cells were harvested and counts were taken.

### Example 3

Approximately 2-3 ml of blood was collected from acute lymphoblastic leukaemia patients (n = 30). Peripheral blood mononuclear cells were separated by Ficoll Hypaque density centrifugation and seeded at a density of 1x10⁵ cells/well in RPMI 1640, supplemented with glutamine, antibiotics and 10 % heat inactivated human AB serum. Cell viability was more than 95 % as checked by their ability to exclude Trypan blue dye (0.2 %). Peripheral blood mononuclear cells were cultured in triplicates (1x10⁵ cells/well) with different concentration of AchatininH (0.05-10µg) in 96 well flat bottom microtitre plates in a total volume of 250 µl at 37°C in a humidified atmosphere at 5% CO₂ and 95 % air for 4 days. Cultures were pulsed, with MTT 4 hours before the termination of culture at 37°C. At the end of the incubation, a solvent DMSO was added to dissolve the purple formazan crystals. The optical density was measured at 560 nm with 690 nm reference in an ELISA reader.

### Example 4

Approximately 2-3 ml of blood was collected from acute lymphoblastic leukaemia patients (n=26). Peripheral blood mononuclear cells were separated by Ficoll Hypaque density centrifugation and seeded at a density of 1x10⁴ cells/well in RPMI 1640, supplemented with glutamine, gentamycin and 10 % heat inactivated human AB serum. Cell viability was more than 95 % as checked by their ability to exclude Trypan blue dye (0.2 %). Peripheral Blood mononuclear cells were cultured in triplicates (1x10⁵ cells/well) with different concentration of AchatininH (0.05-10µg) in 96 well flat bottom microtitre plates in a total volume of 250µl, at 37°C in a humidified atmosphere of 5% CO₂ and 95 % air for 3 days. Culture were pulsed with MTT 4 hours before the termination of culture at 37°C. At the end of the incubation, a solvent DMSO was added to dissolve the purple formazan crystals. The optical density was measured at 560 nm with 690 nm reference in an ELISA reader.

The lymphoproliferation assay carried out according to the process of the present invention (examples 1-4) has been identified as an accurate method for the identification of a new key marker, namely. 9-O-Acetylated Sialoglycoconjugate with the help of a known 9-O-Acetylsialic acid binding lectin. AchatininH useful for the diagnosis of acute lymphoblastic leukaemia (ALL), detection of minimal residual minimal residual disease (MRD) and prediction of relapse and has the following properties.

The level of residual leukaemia cells i.e. minimal residual disease (MRD) was determined by the optimal concentration of AchatininH required for maximal proliferation. This invented process clearly demonstrates that a key marker is expressed at a very high concentration during acute phase, Phase A , as indicated by the low dose of AchatininH (0.19 ± 0.02µg) needed for maximum proliferation of peripheral blood mononuclear cells (Table 1). It showed a decreased expression following treatment, Phase B, as indicated by the distinct increase in the dose of AchatininH ranging from 1.34± 0.13 µg (Table 2). During maintenance therapy, Phase C (Table 3) and follow up. Phase D (Table 4), no blast cell was observed in the peripheral blood indicating normal blood picture. *This invented process still can observe stimulation indicating the presence of some key marker i.e. cell surface receptor - "9 ()-acetylated sialoglycoconjugate" on the surface of peripheral blood mononuclear cells.* The dose of AchatininH required was 5.63± 0.3 µg during maintenance therapy (Table 3) which was not equivalent to normal peripheral blood mononuclear cells. So despite the normal, clinical blood picture, the patients under maintenance therapy and follow up still consist of residual leukaemic cells i.e. minimal residual disease (MRD) (Phase C, D, E in Table 3,4, 5) from the decline in the dose of AchatininH. Five different blood related (NHL, CML, AML, Thalassemmia and Aplastic anemia) served as negative controls showing no cross reactivity with Acute lymphoblastic leukaemia (ALL) by this invented process (Table 6). 25 normal human volunteers of different ages, having different blood groups showed AchatininH dose of 7.92± 0.08µg (Table 7) as compared to Acute lymphoblastic leukaemia (ALL) patients under different phases of treatment. Relapse (Table 5) could be successfully predicted by determining the alteration in maximal lymphoproliferative dose of AchatininH of these patients at different stages of the disease

### RESULTS OF INDIVIDAL PATIENTS:

Study included 192 human blood samples. All these patients were divided according to their duration of treatment.

**TABLE:- 1.**

| **PHASE A** (0-4 weeks treatment/first remission) | | | | |
|---|---|---|---|---|
| Patient No | Sex, Age(Year) | Treatment (weeks). | Peripheral cell blast (%). | Maximal lymphoprolifer ative dose of AchatininH. |
| 1. | M, 6 | 0 | 84 | 0.1 |
| 2. | M, 3.5 | 0 | 30 | 0.1 |
| 3. | M, 5 | 0 | 24 | 0.1 |
| 4. | M, 3 | 1 | 20 | 0.25 |
| 5. | M, 8 | 0 | 60 | 0.1 |
| 6. | M, 5 | 0 | 50 | 0.1 |
| 7. | M, 5 | 2 | 4 | 0.1 |
| 8. | M, 5 | 2 | 25 | 0.25 |
| 9. | M, 5 | 0 | 25 | 0.25 |
| 10. | M, 2.5 | 0 | 80 | 0.5 |
| 11. | M, 2.5 | 0 | 38 | 0.5 |
| 12. | M, 6 | 0 | 64 | 0.1 |
| 13. | F, 5 | 4 | 10 | 0.1 |
| 14. | M, 5 | 1 | 30 | 0.1 |
| 15. | M, 5 | 0 | 38 | 0.1 |
| 16. | M, 3 | 1 | 30 | 0.5 |
| 17. | M, 7 | 4 | 10 | 0.5 |
| 18. | M, 6 | 4 | 3 | 0.1 |
| 19. | M, 6 | 0 | 84 | 0.5 |
| 20. | M, 5 | 2 | 7 | 0.25 |
| 21. | M, 3.5 | 1 | 40 | 0.25 |
| 22. | M, 5 | 2 | 30 | 0.5 |
| 23. | M, 6 | 0 | 80 | 0.1 |
| 24. | F, 13 | 0 | 60 | 0.25 |
| 25. | F, 7.5 | 0 | 34 | 0.1 |
| 26. | M, 3 | 1 | 49 | 0.1 |
| 27. | M, 5 | 0 | 54 | 0.1 |
| 28. | M, 3.5 | 1 | 40 | 0.25 |
| 29. | F, 8 | 0 | 60 | 0.1 |
| 30. | F, 7.5 | 0 | 38 | 0.25 |
| 31. | M, 8 | 0 | 80 | 0.1 |
| 32. | M, 3.5 | 4 | 40 | 0.1 |
| 33. | M, 6 | 0 | 58 | 0.1 |
| 34. | M, 1.5 | 0 | 95 | 0.1 |
| 35. | F, 7.5 | 2 | 25 | 0.25 |
| 36. | M, 4 | 1 | 22 | 0.1 |
| 37. | M, 6 | 0 | 63 | 0.1 |
| 38. | M, 3.5 | 0 | 74 | 0.1 |
| 39. | M, 3.5 | 1 | 12 | 0.25 |
| 40. | M, 4 | 2 | 42 | 0.1 |
| 41. | M, 3.5 | 4 | 10 | 0.1 |
| 42. | M, 5 | 4 | 7 | 0.25 |
| 43. | M, 5 | 0 | 81 | 0.1 |
| 44. | M, 5 | 0 | 33 | 0.1 |
| 45. | M, 5 | 4 | 5 | 0.5 |
| 46. | F, 7 | 0 | 55 | 0.1 |
| 47. | M, 7.5 | 0 | 57 | 0.1 |
| 48. | F, 7 | 0 | 39 | 0.1 |
| 49. | F, 7 | 0 | 63 | 0.1 |
| 50. | F, 7 | 0 | 51 | 0.1 |
| 51. | M, 4 | 0 | 90 | 0.1 |
| 52. | F, 5 | 0 | 39 | 0.1 |
| 53. | M, 6 | 1 | 51 | 0.1 |
| 54. | M, 3 | 0 | 60 | 0.1 |
| 55. | M, 8 | 1 | 57 | 0.1 |
| 56. | M, 12 | 0 | 73 | 0.1 |
| 57. | F, 9 | 3 | 44 | 0.1 |
| 58. | M, 5 | 2 | 29 | 0.25 |
| 59. | M, 2.5 | 0 | 22 | 0.1 |
| 60. | M, 6 | 0 | 57 | 0.1 |
| 61. | F, 5 | 0 | 82 | 0.1 |
| 62. | M, 7 | 0 | 53 | 0.1 |
| 63. | M, 5 | 4 | 10 | 1.0 |
| 64. | M, 7 | 4 | 9 | 0.25 |
| 65. | M, 2 | 0 | 79 | 0.1 |
| **Result** :- Phase A comprised of 65 Acute lymphoblastic leukaemia (ALL) patients. The mean (X± SE) of the 'maximal lymphoproliferative dose' of AchatininH was 0.19± 0.019ug /1x10⁵ cells reflecting a significantly high level of *9 O- acetylated sialoglycoconjugate" on the surface of peripheral blood mononuclear cells* during Phase A. A blast cell % of 45.1± 2.97 was observed in Phase A. Therefore the 'maximal lymphoproliferative dose of AchatininH' bears an inverse relationship with the expression of *9 O- acetylated sialoglycoconjugate" on the surface of peripheral blood mononuclear cells* (PBMC). This indicates that the lesser the 'maximal lymphoproliferative dose of AchatininH' the more is the expression of *9 O- acetylated sialoglycoconjugate" on the surface of peripheral blood mononuclear cells*. The level of *9 O- acetylated sialoglycoconjugate*" *on the surface of peripheral blood mononuclear cells*, as indicated by the 'maximal lymphoproliferative dose of AchatininH', serves as a direct reflection of % blast cells i.e. acuteness of the disease. Thus the phase of treatment of Acute lymphoblastic leukaemia (ALL) patients could be diagnosed by this invented method. | | | | |

**TABLE :- 2**

| **PHASE B** (4-8 weeks treatment/early intensification). | | | | |
|---|---|---|---|---|
| Patient No. | Sex, Age (years). | Treatment (weeks). | Peripheral blast cell (%). | Maximal lymphoprolifer ative dose of AchatininH. |
| 1. | M, 6 | 5 | 0 | 2.0 |
| 2. | M, 5 | 6 | 2 | 0.25 |
| 3. | M, 8 | 7 | 0 | 1.0 |
| 4. | M, 7 | 8 | 2 | 2.0 |
| 5. | M, 5 | 6 | 2 | 2.0 |
| 6. | F, 7 | 4 | 2 | 0.5 |
| 7. | M, 12 | 5 | 10 | 1.0 |
| 8. | M, 6 | 6 | 0 | 1.0 |
| 9. | M, 5 | 7 | 3 | 1.0 |
| 10. | M, 12 | 8 | 0 | 2.0 |
| 11. | M, 12 | 5 | 0 | 1.0 |
| 12. | M, 5 | 6 | 3 | 2.0 |
| 13. | M, 5 | 7 | 0 | 2.0 |
| 14. | M, 3 | 5 | 2 | 2.0 |
| 15. | F, 5 | 5 | 0 | 1.0 |
| 16. | M, 14 | 5 | 0 | 1.0 |
| 17. | M, 4 | 7 | 2 | 1.0 |
| 18. | M, 5 | 5 | 2 | 1.0 |
| 19. | M, 5 | 6 | 2 | 2.0 |
| 20. | M, 6 | 6 | 0 | 0.5 |
| 21. | M, 7 | 4 | 0 | 0.5 |
| 22. | M, 6 | 4 | 3 | 3.0 |
| 23. | M, 7 | 4 | 0 | 2.0 |
| 24. | M, 5 | 5 | 0 | 0.5 |
| 25. | M, 7 | 7 | 0 | 1.0 |
| 26. | M, 14 | 8 | 0 | 1.0 |
| 27. | F, 11 | 8 | 0 | 2.0 |
| **Result** :- Phase B comprised of 27 ALL patients and the 'maximal lymphoproliferative dose of AchatininH' (X ± SE) increased to 1.34 ± 0.13ug reflecting a decline in the expression of the key marker, namely, *9 O- acetylated sialoglycoconjugate" on the surface of peripheral blood mononuclear cells.* The difference between the 'maximal lymphoproliferative dose of AchatininH' in Phase A and Phase B was statistically significant (p<0.0005). The mean blast cell % of 1.3 ± 0.4 in Phase B reiterates a relationship between the expression of *9 O- acetylated sialoglycoconjugate" on the surface of peripheral blood mononuclear cells* and the acuteness of the disease. Thus chemosensitivity of ALL patients could be detected by this invented process. | | | | |

**TABLE :- 3**

| **PHASE C** (8 weeks-2.5 years treatment/ maintenance therapy). | | | |
|---|---|---|---|
| Patient No. | Sex, Age (years). | Treatment (weeks). | Maximal lymphoproliferative dose of AchatininH. |
| 1. | F, 11 | 12 | 2.0 |
| 2. | M, 5 | 8 | 3.0 |
| 3. | M, 6 | 7 | 4.0 |
| 4. | M, 5 | 24 | 4.0 |
| 5. | M, 8 | 19 | 6.0 |
| 6. | M, 8 | 21 | 6.0 |
| 7. | F, 11 | 12 | 2.0 |
| 8. | M, 6 | 24 | 6.0 |
| 9. | M, 5 | 24 | 3.0 |
| 10. | M, 8 | 19 | 6.0 |
| 11. | F, 10 | 35 | 6.0 |
| 12. | M, 5 | 12 | 6.0 |
| 13. | M, 7.5 | 34 | 6.0 |
| 14. | F, 12 | 42 | 6.0 |
| 15. | M, 12 | 9 | 2.0 |
| 16. | M, 14 | 47 | 8.0 |
| 17. | M, 8 | 22 | 8.0 |
| 18. | M, 5 | 24 | 6.0 |
| 19. | M, 7 | 32 | 4.0 |
| 20. | F, 14 | 48 | 6.0 |
| 21. | M, 6 | 60 | 6.0 |
| 22. | M, 6 | 50 | 6.0 |
| 23. | M, 6 | 62 | 6.0 |
| 24. | M, 7 | 28 | 6.0 |
| 25. | M, 7 | 44 | 8.0 |
| 26. | M, 8 | 48 | 8.0 |
| 27. | M, 12 | 51 | 6.0 |
| 28. | M, 12 | 51 | 8.0 |
| 29. | M, 5 | 11 | 3.0 |
| 30. | M, 5 | 33 | 8.0 |
| 31. | F, 5 | 44 | 8.0 |
| 32. | M, 14 | 23 | 4.0 |
| 33. | M, 14 | 15 | 4.0 |
| 34. | M, 7 | 9 | 8.0 |
| 35. | M, 8 | 22 | 8.0 |
| **Result :-** Phase C included 35 ALL patients. The mean (X ± SE) of the 'maximal lymphoproliferative dose of AchatininH' is 5.63 ± 0.30/1X10⁵ cells. Hence the 'maximal lymphoproliferative dose of AchatininH' shows a progressive increase with treatment reflecting a decline in the expression of *9 O- acetylated sialoglycoconjugate" on the surface of peripheral blood mononuclear cells* with treatment. However, it must be noted that although no blast cells could be detected by available methods, the 'maximal lymphoproliferative dose of AchatininH', even in maintenance therapy, was significantly less as compared to normal donors (Table 7). Thus the *9 O- acetylated sialoglycoconjugate" on the surface of peripheral blood mononuclear cells* expression in maintenance therapy is significantly higher than normal donors. The *9 O- acetylated sialoglycoconjugate" on the surface of peripheral blood mononuclear cells* expression serves as direct reflection of blast cell % and minimal residual disease (MRD) can be detected by this invented process. | | | |

**TABLE: 4**

| **PHASE D** (patients followed up after completion of maintenance therapy). | | | |
|---|---|---|---|
| Patient No. | Sex, Age (years). | Treatment (weeks). | Maximal lymphoproliferative dose of AchatininH. |
| 1. | M, 5 | 88 | 6.00 |
| 2. | M, 6.5 | 96 | 6.00 |
| 3. | M, 8 | 96 | 4.00 |
| 4. | M, 5 | 96 | 6.00 |
| 5. | M, 5 | 136 | 6.00 |
| 6. | M, 6.5 | 126 | 6.00 |
| 7. | M, 5 | 120 | 6.00 |
| 8. | M, 8 | 150 | 6.00 |
| 9. | M, 8 | 136 | 6.00 |
| 10. | M, 8 | 192 | 8.00 |
| 11. | M, 7 | 208 | 8.00 |
| **Results** :- Phase D included 11 patients who were followed up after completion of maintenance therapy. The mean (X ± SE) of the 'maximal lymphoproliferative dose of AchatininH' was 6.18 ± 0.32ug/1X10⁵ cells, which was significantly less in comparison to normal donors (Table 7) reflecting the persistence of the blast cells by this method. Thus MRD could be detected by this invented process. | | | |

**TABLE :- 5**

| **RELAPSE CASES** (patients for which relapse could be successfully predicted by our invented assay). | | | | |
|---|---|---|---|---|
| Patient No. | Sex, Age (Years). | Time at which relapse occurred. | Maximal lymphoproliferative dose of AchatininH _{H} . | Remarks. |
| 1. | F, 7.5 | One Year after the completion of maintenance therapy | 0.25 | Relapse predicted by the invented patent. |
| 2. | F, 7.5 | -do- | 0.25 | -do- |
| 3. | M, 7 | During maintenance therapy. | 1.0 | -do- |
| 4. | M, 5 | One Year after maintenance therapy | 0.1 | -do- |
| 5. | M, 14 | Six months after maintenance therapy. | 1.0 | -do- |
| 6. | M, 5 | After maintenance therapy. | 2.0 | -do- |
| 7. | M, 7 | During maintenance therapy. | 0.25 | -do- |
| 8. | M, 5 | -do- | 3.0 | -do- |
| **Result :-** The decreased in 'maximal lymphoproliferative dose of AchatininH' indicates the reexpression of 9 O- acetylated sialoglycoconjugate" on the surface of peripheral blood mononuclear cells. The expression of 9 O-acetylated sialoglycoconjugate" is more as compared to that maintenance therapy (Table 3) and follow-up (Table 4). Thus decreased in 'maximal lymphoproliferative dose of AchatininH' indicates the reexpression of 9 O-acetylated sialoglycoconjugate" on the surface of peripheral blood mononuclear cells and could predict relapse in these patients. Our prediction correlated well with the clinical observation. | | | | |

**TABLE :- 6.**

| **Results of patients studied for cross reactivity :-** | | | |
|---|---|---|---|
| Patient No. | Sex, Age (Years). | Disease. | Response to AchatininH. |
| 1. | M, 12 | Thalassemmia | 8.00 |
| 2. | M, 12 | -do- | 8.00 |
| 3. | M, 5 | -do- | 8.00 |
| 4. | M, 5 | -do- | 8.00 |
| 5. | M, 10 | NHL | 8.00 |
| 6. | M, 7 | AML | No response |
| 7. | M, 7 | AML | -do- |
| 8. | F, 11 | NHL | -do- |
| 9. | M, 38 | CML | -do- |
| 10 | M, 35 | CML | -do- |
| 11. | M, 42 | CML | -do- |
| 12. | F, 40 | CML | -do- |
| 13. | M, 33 | CML | -do- |
| 14. | F, 9 | AML | -do- |
| 15. | M, 5 | AML | -do- |
| 16. | M, 48 | CML | -do- |
| 17. | M, 7 | Aplastic anemia | -do- |
| 18. | M, 5 | -do- | -do- |
| 19. | F, 8 | Thalassemmia | 8.00 |
| 20. | M, 12 | NHL | 8.00 |
| 21. | M, 8 | NHL | 8.00 |
| **Results** :- In Thalassemmic and NHL (Non Hodgkin's Lymphoma) patients the maximal lymphoproliferative dose of AchatininH' was similar to normal human donors (Table 7). No lymphoproliferation was observed in CML (Chronic Myelogenous Leukemia), AML (Acute Myelogenous Leukemia) and Aplastic Anemia patients. Thus patients of other diseases (NHL, CML, AML, Thalassemmia and Aplastic Anemia) served as negative controls showing no cross reactivity with ALL by this invented process. | | | |

**TABLE :- 7**

| **Response of the normal donors :-** | | | |
|---|---|---|---|
| Normal donors. | Sex, Age (Years). | Blood Groups. | Maximal lymphoproliferative dose of AchatininH. |
| 1. | F, 24 | A+ | 8.00 |
| 2. | M, 10 | ND. | 8.00 |
| 3. | M, 15 | -do- | 8.00 |
| 4. | F, 24 | A+ | 8.00 |
| 5. | M, 23 | B ⁻ | 8.00 |
| 6. | M, 25 | O+ | 8.00 |
| 7. | F, 41 | A+ | 8.00 |
| 8. | M, 36 | O+ | 8.00 |
| 9. | M, 5 | ND | 6.00 |
| 10. | F, 25 | A+ | 8.00 |
| 11. | F, 25 | -do- | 8.00 |
| 12. | F, 41 | -do- | 8.00 |
| 13. | F, 25 | -do- | 8.00 |
| 14. | F, 25 | -do- | 8.00 |
| 15. | F, 41 | -do- | 8.00 |
| 16. | M, 12 | ND | 8.00 |
| 17. | M, 38 | O+ | 8.00 |
| 18. | F, 24 | ND | 8.00 |
| 19. | M, 11 | ND | 8.00 |
| 20. | M, 15 | ND | 8.00 |
| 21. | M, 24 | ND | 8.00 |
| 22. | M, 26 | ND | 8.00 |
| 23. | M, 9 | ND | 8.00 |
| 24. | F, 41 | A+ | 8.00 |
| 25. | F, 26 | A+ | 8.00 |
| **Results** :- 25 normal human volunteers of different ages , having different blood groups were included in study. The mean (X ± SE) of the 'maximal lymphoproliferative dose of AchatininH' was 7.92 ± 0.08ug/1X10⁵ cell, reflecting a very low expression of 9-O Acetyl Sialo Glyco conjugate on PBMC surface of normal donors as compared to ALL patients under different phases of treatment. | | | |

### The salient advantages of the proposed invention are

(a) A simple, rapid and easy, method to perform employing peripheral blood (2-3 ml of blood per patients) It requires peripheral blood as the starting material in place of bone marrow.
(b) The abundant availability of *Achatina Fulica* snail and single step purification with high yield (6%) and its restricted specificity (Mandal C, Basu S. Biochem Biophys Res Commun 1987 ; 148 : 795-801, Sen G. Mandal C. Carbohydrate Research 1995 ; 268 : 115-125 ) makes it, useful tool for biochemical analysis. The lectin also has long term stability for > 2years and can be stored at 6°C-10°C in a filter sterilized condition at a concentration of 1mg/ml.
(c) It can detect leukaemic blast cells in peripheral blood in the acute phase of the disease. More importantly, it can detect residual leukaemic i.e. blast cells in peripheral blood even when the bone marrow picture is apparently normal (less than 5% blast cells still present, but not detectable by conventional methods), and hence can be used for detection of minimal residual disease (MRD);
(d) The assay can assess response of ALL patients to chemotherapy and hence can also be used for determining drug sensitivity and the duration of treatment;
(e) If performed with peripheral blood of follow-up patients, it can predict relapses;
(f) It reflects the expression of a common maker, a 9-OAcSG, in ALL patients of different linages i.e. it can be used for different types of ALL (T cell ALL, B cell ALL and other types );
(g) It can distinguish between different stages of acute lymphoblastic leukaemia which correlate well with the clinical status of the disease;
(h) It detects the extent to which the patient has responded to chemotherapy;
(i) Detects the percentage of recovery of individual ALL patients in response to chemotherapy
(j) It shows no cross reactive response with other hematological disorder;
(k) It requires no sophisticated instrument and minimum processing; and
(i) Owing to low cost (∼ Rs 400) involved in this invention, it can be widely applied in any clinical laboratories of developing countries like India.
However, the lymphoproliferative assay by conventional ³H-thymidine uptake has some disadvantages as it is a radiometric and it is necessary to harvest and wash the cells which increases the processing time.
In comparison, the colorimetric assay employing 3-(4,5-dimethylthiazole-2-yl)-2,5-diphenyltetrazolium bromide (MTT) is non radiometric and rapid since it is not necessary to harvest and wash the cells and the enzyme activity can be directly read on an automatic spectrophotometer or an ELISA plate reader (Mosman., 1983 ; Denizot., 1986 ; Tada., 1986 ; Kaspers., 1995).
However, owing to its dependence both on the number of cells and mitochondrial activity per cell type, each assay parameter was standardized to obtain optimal sensitivity and results compared with that of ³H-Thymidine uptake, the MTT assay can serve as an alternative readout for cellular growth and survival of lymphocytes in microcultures.
Other applications of the lectin:

The lectin Achatinin-H, is a unique probe for identification of the cell surface marker "9-O-Acetylated sialoglycoconjugate". It has been utilised in two completely different systems, the common feature being the cell surface marker "9-O-Acetylated sialoglycoconjugate". The two diseases in question are (i) visceral leishmaniasis and (ii) acute lymphoblastic leukemia.

Clinically, the two disease processes are completely different from each other and are easily distinguishable as also the mode of treatment are totally different. With regard to the invention, the important differences are as follows:
1. In the proposed invention the diagnosis of visceral leishmaniasis is based on the presence of the biomarker "9-O-Acetylated sialoglycoconjugate" on erythrocytes whereas in acute lymphoblastic leukemia it is based on the presence of the biomarker "9-O-Acetylated sialoglycoconjugate" on peripheral blood mononuclear cells.
2. In the proposed invention the diagnosis of visceral leishmaniasis is based on the agglutination of erythrocytes using the lectin Achatinin-H whereas in acute lymphoblastic leukemia it is based on the lymphoproliferative assay using the lectin Achatinin-H.
3. The diagnosis of visceral leishmaniasis is based on the naked eye evaluation of agglutination of erythrocytes using the lectin Achatinin-H whereas in acute lymphoblastic leukemia it is based on the lymphoproliferative assay using the lectin Achatinin-H which is measured either by radiometric or colorometric assay.
4. The haemagglutination assay is positive only during the acute phase of the disease in visceral leishmaniasis prior to treatment whereas in acute lymphoblastic leukemia the detection of minimal residual disease and prediction of relapse the proposed invention is of utmost importance after initial phase of chemotherapy.

## Claims

1. A method for the detection of minimal residual disease and prediction of relapse by identifying a 9-O-acetylated sialoglycoconjugate key marker on the surface of peripheral blood mononuclear cells of acute lymphoblastic leukaemia patients, said method comprising treating blood, collected from acute lymphoblastic leukaemia patients at different clinical states of the disease, by separating peripheral blood mononuclear cells by centrifugation; culturing said mononuclear (PBM) cells in tissue culture medium with a 9-O-acetylated sialic binding lectin; detecting the stimulated PBM cells and thereby diagnosing the disease.

2. A method as claimed in claim 1 wherein the cultures were pulsed with 3-(4,5-dimethylthiazole-2-yl)-2,5-diphenyltetrazolium bromide (MTT) for 4 hours before the termination of culture.

3. A method as claimed in claim 2 wherein the termination takes place at 37°C.

4. A method as claimed in claims 2 or 3, wherein DMSO is added to dissolve the purple formazan crystals at the end of the incubation.

5. A method as claimed in claim 1, wherein the level of residual leukaemia cells (MRD) is determined by the optimal concentration of 9-O-acetyl sialic binding lectin required for maximal proliferation.

6. A method as claimed in claim 1, wherein the whole blood is used for peripheral blood mononuclear cells operation.

7. A method as claimed in claim 1, wherein the 9-O-acetyl sialic binding lectin is Achatinin H.

8. A method as claimed in claim 7, wherein the AchatininH is purified from the hemolymph of *AchatininH Fulica* snail.

9. A method as claimed in claim 7, wherein the concentration of AchatininH is used in the range of 0.05-10µg for stimulation of peripheral blood mononuclear cells.

10. A method as claimed in claim 1, wherein said culture medium is selected from a group comprising DMEM, foetal calf serum, RPMI 1640, supplemented with glutamine, antibiotics and 10% heat inactivated human AB serum.

11. A method as claimed in claim 1, wherein culturing of the peripheral blood mononuclear cells is performed in sterile, microtitre plates, or test tubes.

12. A method as claimed in claim 2, wherein the peripheral blood mononuclear cells are cultured in tissue culture medium with AchatininH in 96 well flat bottom microtitre plates at 37°C in a CO² incubator for 4 days.

13. A method as claimed in claim 1 wherein 0.25µl to 0.5 ml of a total reaction volume is used to culture peripheral blood mononuclear cells.

14. A method as claimed in claim 1, wherein said peripheral blood mononuclear cells are cultured at 37°C in the presence of AchatininH.

15. A method as claimed in claim 1, wherein said peripheral blood mononuclear cells are cultured in the presence of AchatininH in a humidified atmosphere of 5% CO₂ and 95 % air.

16. A method as claimed in claim 1 wherein a culture time of 4 days is used for maximum stimulation.

17. A method as claimed in claim 1 wherein peripheral blood mononuclear cells concentration of 1x 10⁵ cells/well are used.

18. A method as claimed in claim 1, wherein the cultures are pulsed with 37 kBq (1µCi) of ³H thymidine or 1.85 kBq (0.05µCi) of ³H thymidine.

19. A method as claimed in claim 1, wherein 37 kBq (1µCi) of 3H thymidine is added 18 hours before the termination of culture.

20. A method as claimed in claim 1, wherein the detection of stimulated peripheral blood mononuclear cells comprises harvesting the cells and counting such cells.

21. A method as claimed in claim 1, wherein the optical density is measured at 560 nm with 690 nm reference in an ELISA reader.

22. A method as claimed in claim 4, wherein the amount of formazan produced which is proportional to the number of viable cells after cell proliferation is estimated colorimetrically.

23. A method as claimed in claim 1, wherein the dose of AchatininH (µg/1x10⁵ cells) needed for maximum proliferation of peripheral blood mononuclear cells is calculated.

24. A method as claimed in claim 1, wherein the dose of AchatininH(µg/1x10⁵ cells) need for maximum proliferation of peripheral blood cells been correlated with the status of the disease, increasing with progress of chemotherapy.

25. A method as claimed in claim 1, wherein the level of minimal residual leukaemia cells (MRD) is determined by the optimal concentration of AchatininH required for maximal proliferation.

## Patentansprüche

1. Verfahren für den Nachweis einer minimalen Restkrankheit und zur Vorhersage eines Rezidivs durch Identifizierung eine 9-O-acetylierten Sialylglycokonjugat Schlüsselmarkierung auf der Oberfläche mononukleärer Zellen des peripheren Bluts von Patienten mit akuter lymphatischer Leukämie, wobei das Verfahren die Behandlung von Blut, gesammelt von Patienten mit akuter lymphatischer Leukämie zu verschiedenen klinischen Stadien der Erkrankungen, durch Abtrennung der mononukleären Zellen des peripheren Bluts durch Zentrifugation; die Kultivierung dieser mononukleären (PBM) Zellen in Zellkulturmedium mit einem 9-O-acetylierten Sialin bindenden Lektin; Nachweis der stimulierten PBM-Zellen und dadurch Diagnostizierung der Krankheit umfasst.

2. Verfahren nach Anspruch 1, wobei die Kulturen mit 3-(4,5-Dimethylthiazol-2-yl)-2,5-diphenyltetrazoliumbromid (MTT) für 4 Stunden vor der Beendigung der Kultivierung gepulst werden.

3. Verfahren nach Anspruch 2, wobei die Beendigung bei 37°C stattfindet.

4. Verfahren nach den Ansprüchen 2 oder 3, wobei DMSO zugegeben wird, um die purpurfarbenen Formazankristalle am Ende der Inkubation zu lösen.

5. Verfahren nach Anspruch 1, wobei der Anteil der restlichen Leukämiezellen (MRD) durch die für eine maximale Proliferation benötigte optimale Konzentration von 9-O-Acetylsialin bindenden Lektin bestimmt wird.

6. Verfahren nach Anspruch 1, wobei das Vollblut für den Vorgang mit den mononukleären Zellen des peripheren Bluts verwendet wird.

7. Verfahren nach Anspruch 1, wobei das 9-O-Acetylsialin bindende Lektin Achatinin-H ist.

8. Verfahren nach Anspruch 7, wobei das Achatinin-H aus der Hämolymphe der Schnecke *Achatina fulica* gereinigt wird.

9. Verfahren nach Anspruch 7, wobei Achatinin-H in einer Konzentration von 0,05-10µg zur Stimulation der mononukleären Zellen des peripheren Bluts verwendet wird.

10. Verfahren nach Anspruch 1, wobei das Kulturmedium mit fötalen Kälberserum ergänztes DMEM, oder mit Glutamin, Antibiotika und 10% hitzinaktivierten menschlichen AB-Serum ergänztes RPMI 1640 ist.

11. Verfahren nach Anspruch 1, wobei die Kultivierung der mononukleären Zellen des peripheren Bluts in sterilen Mikrotiterplatten oder Teströhrchen erfolgt.

12. Verfahren nach Anspruch 2, wobei die mononukleären Zellen des peripheren Bluts in Gewebekulturmedium mit Achatinin-H in Mikrotiterplatten mit 96 Vertiefungen und flachem Boden bei 37°C in einem CO₂-Brutschrank für 4 Tage kultiviert werden.

13. Verfahren nach Anspruch 1, wobei ein Gesamtreaktionsvolumen von 0,25µl bis 0,5ml zur Kultivierung der mononukleären Zellen des peripheren Bluts verwendet wird.

14. Verfahren nach Anspruch 1, wobei die mononukleären Zellen des peripheren Bluts bei 37°C in Anwesenheit von Achatinin-H kultiviert werden.

15. Verfahren nach Anspruch 1, wobei die mononukleären Zellen des peripheren Bluts in Anwesenheit von AchatininH in einer feuchten Atmosphäre mit 5% CO₂ und 95% Luft kultiviert werden.

16. Verfahren nach Anspruch 1, wobei eine Kultivierungsdauer von 4 Tagen für die maximale Stimulation verwendet wird.

17. Verfahren nach Anspruch 1, wobei mononukleäre Zellen des peripheren Bluts in einer Zelldichte von 1x10⁵ Zellen/Vertiefung verwendet werden.

18. Verfahren nach Anspruch 1, wobei die Kulturen mit 37 kBq (1µCi) ³H-Thymidin oder 1,85 kBq (0,05µCi) ³H-Thymidin gepulst werden.

19. Verfahren nach Anspruch 1, wobei 37 kBq (1µCi) ³H-Thymidin 18 Stunden vor der Beendigung der Kultur zugegeben wird.

20. Verfahren nach Anspruch 1, wobei der Nachweis der stimulierten mononukleären Zellen des peripheren Bluts die Ernte der Zellen und das Zählen solcher Zellen umfasst.

21. Verfahren nach Anspruch 1, wobei die optische Dichte bei 560 nm mit einem Referenzwert bei 690 nm in einem ELISA-Lesegerät gemessen wird.

22. Verfahren nach Anspruch 4, wobei die erzeugte Formazanmenge, welche proportional zu der Anzahl der lebensfähigen Zellen nach der Zellproliferation ist, kolorimetrisch bestimmt wird.

23. Verfahren nach Anspruch 1, wobei die für die maximale Proliferation der mononukleären Zellen des peripheren Bluts benötigte Dosis an Achatinin-H (µg/1x10⁵ Zellen) berechnet wird.

24. Verfahren nach Anspruch 1, wobei die für die maximale Proliferation der mononukleären Zellen des peripheren Bluts benötigte Dosis an Achatinin-H (µg/1x10⁵ Zellen) mit dem Krankheitszustand korreliert und mit fortschreitender Chemotherapie ansteigt.

25. Verfahren nach Anspruch 1, wobei der Anteil der minimalen Restleukämiezellen (MRD) durch die optimale Konzentration des für maximale Proliferation benötigten Achatinin-H bestimmt wird.

## Revendications

1. Procédé de détection d'une maladie résiduelle minimale et de prévision d'une récidive par l'identification d'un marqueur clé 9-O-acétylsialoglycoconjugué sur la surface de cellules mononucléées du sang périphérique de patients atteints de leucémie lymphoblastique aiguë, ce procédé comprenant le traitement de sang prélevé chez des patients atteints de leucémie lymphoblastique aiguë à différents stades cliniques de la maladie, la séparation des cellules mononucléées du sang périphérique par centrifugation, la culture de ces cellules mononucléées (PBMC) dans un milieu de culture de tissus avec une lectine se liant à l'acide sialique 9-O-acétylé, la détection des cellules PBMC stimulées, et ainsi le diagnostic de la maladie.

2. Procédé selon la revendication 1, dans lequel les cultures ont été soumises à des impulsions avec du bromure de 3-(4,5-diméthylthiazol-2-yl)-2,5-diphényltétrazolium (MTT) pendant 4 heures avant l'arrêt de la culture.

3. Procédé selon la revendication 2, dans lequel l'arrêt a lieu à 37°C.

4. Procédé selon les revendications 2 ou 3, dans lequel on ajoute du DMSO pour dissoudre les cristaux de formazane pourpres à la fin de l'incubation.

5. Procédé selon la revendication 1, dans lequel on détermine le niveau de cellules leucémiques résiduelles (MRD) par la concentration optimale de lectine se liant à l'acide 9-O-acétylsialique nécessaire pour une prolifération maximale.

6. Procédé selon la revendication 1, dans lequel on utilise le sang total pour le traitement des cellules mononucléées du sang périphérique.

7. Procédé selon la revendication 1, dans lequel la lectine se liant à l'acide 9-O-acétylsialique est l'achatinine H.

8. Procédé selon la revendication 7, dans lequel l'achatinine H est purifiée à partir de l'hémolymphe de l'escargot *AchatininH Fulica*.

9. Procédé selon la revendication 7, dans lequel on utilise une concentration d'achatinine H comprise entre 0,05 et 10 µg pour la stimulation des cellules mononucléées du sang périphérique.

10. Procédé selon la revendication 1, dans lequel ledit milieu de culture est du DMEM complété avec du sérum de veau foetal ou du RPMI 1640 complété avec de la glutamine, des antibiotiques et 10 % de sérum AB humain inactivé à la chaleur.

11. Procédé selon la revendication 1, dans lequel la culture des cellules mononucléées du sang périphérique s'effectue dans des plaques de microtitrage stériles ou des tubes à essai.

12. Procédé selon la revendication 2, dans lequel on cultive les cellules mononucléées du sang périphérique dans un milieu de culture de tissus avec de l'achatinine H dans des plaques de microtitrage à fond plat de 96 puits à 37°C dans un incubateur à CO₂ pendant 4 jours.

13. Procédé selon la revendication 1, dans lequel on utilise 0,25 µl à 0,5 ml de volume de réaction total pour la culture des cellules mononucléées du sang périphérique.

14. Procédé selon la revendication 1, dans lequel on cultive lesdites cellules mononucléées du sang périphérique à 37°C en présence d'achatinine H.

15. Procédé selon la revendication 1, dans lequel on cultive lesdites cellules mononucléées du sang périphérique en présence d'achatinine H dans une atmosphère humidifiée à 5 % de CO₂ et 95 % d'air.

16. Procédé selon la revendication 1, dans lequel on utilise un temps de culture de 4 jours pour une stimulation maximale.

17. Procédé selon la revendication 1, dans lequel on utilise une concentration de cellules mononucléées du sang périphérique de 1 × 10⁵ cellules/puits.

18. Procédé selon la revendication 1, dans lequel on soumet les cultures à des impulsions avec 37 kBq (1 µCi) de ³H-thymidine ou 1,85 kBq (0,05 µCi) de ³H-thymidine.

19. Procédé selon la revendication 1, dans lequel on ajoute 37 kBq (1 µCi) de ³H-thymidine 18 heures avant l'arrêt de la culture.

20. Procédé selon la revendication 1, dans lequel la détection des cellules mononucléées du sang périphérique stimulées comprend le recueil des cellules et le comptage de ces cellules.

21. Procédé selon la revendication 1, dans lequel on mesure la densité optique à 560 nm avec une référence de 690 nm dans un lecteur d'ELISA.

22. Procédé selon la revendication 4, dans lequel on estime par colorimétrie la quantité de formazane produit, qui est proportionnelle au nombre de cellules viables après la prolifération des cellules.

23. Procédé selon la revendication 1, dans lequel on calcule la dose d'achatinine H (µg/l × 10⁵ cellules) nécessaire pour une prolifération maximale des cellules mononucléées du sang périphérique.

24. Procédé selon la revendication 1, dans lequel on établit une corrélation entre la dose d'achatinine H (µg/l × 10⁵ cellules) nécessaire pour une prolifération maximale des cellules mononucléées du sang périphérique, augmentant avec l'avancement de la chimiothérapie, et l'état de la maladie.

25. Procédé selon la revendication 1, dans lequel on détermine le niveau de cellules leucémiques résiduelles minimales (MRD) par la concentration optimale d'achatinine H nécessaire pour une prolifération maximale.
